# EUROPÄISCHE PATENTANMELDUNG

(11) **EP 1 059 095 A1**
(43) Veröffentlichungstag der Anmeldung: **13.12.2000**
(21) Anmeldenummer: 00107423.6
(22) Anmeldetag: 06.04.2000
(51) Int. Cl.: A61M 11/00, B05B 11/02, A61K 31/485, A61K 9/12

(54) **Dosiervorrichtung**

(30) Priorität: 11.05.1999 DE 29908435 U
(71) Anmelder: Krewel Meuselbach GmbH, 53775 Eitorf (DE)
(72) Erfinder: Schierstedt, Dieter, Dr., 53757 St. Augustin (DE); Joos, Karl-Heinz, Dr., c/o Krewel Meuselbach GmbH, 53783 Eitorf (DE)
(74) Vertreter: Jönsson, Hans-Peter, Dr.Dipl.-Chem.

(57) **Zusammenfassung**

Gegenstand der vorliegende Erfindung ist eine Dosiervorrichtung für wässrig ethanolische Lösungen von Tilidin.

Die Dosiervorrichtung für wässrig/ethanolische Lösungen von Tilidin umfasst eine metallische Oberflächen aufweisende Pumpdosiereinrichtung (1) zur Tropfengenauen Dosierung der Lösung aus einem Vorlagebehälter, wobei die Lösung Tilidin in Form eines Salzes in einer Menge von 30 bis 100 mg pro ml, Naloxon in Form eines Salzes in einer Menge von 2,5 bis 7 mg pro ml und Ethanol in einer Menge von wenigstens 5 Vol.-%, bezogen auf die Gesamtlösung bis zur Löslichkeitsgrenze des Tilidins bei Raumtemperatur, enthält.

## Beschreibung

Gegenstand der vorliegende Erfindung ist eine Dosiervorrichtung für wässrig ethanolische Lösungen von Tilidin.

Tilidin, [(+/-)-Ethyl-(trans-2-dimethylamino-phenyl-3-cyclohexan-trans-1-carboxylat)] ist ein entfernt mit Morphin verwandtes Opioidanalgetikum, das enteral rasch resorbiert wird und sich dementsprechend zur Schmerztherapie hervorragend eignet.

In der EP 0 665 830 B1 wird beschrieben, daß vornehmlich Salze des basischen Wirkstoffs in Frage kommen, da die Base als solche keine ausreichende Stabilität über einen längeren Zeitraum aufweist. Es wird weiterhin festgehalten, daß alle Anstrengungen, brauchbare feste retardierte Arzneimittelformen des Wirkstoffs Tilidin bereitzustellen, an Stabilitätsproblemen gescheitert sind.

Tilidin wird im Handel üblicherweise zusammen mit einem Morphinantagonisten, beispielsweise Naloxon-Hydrochlorid eingesetzt.

Wässrig ethanolische Lösungen von Tilidin sind seit langem bekannt und im Handel vielfach erhältlich, wie ein Blick in die Rote Liste, Editio Kantor Verlag für Medizin und Naturwissenschaften GmbH, 1999 zeigt. In der eingangs erwähnten EP 0 665 830 B1 wird daraufhingewiesen, daß sich als Salz für stabile flüssige Zubereitungen in der Praxis bisher nur das Hydrochlorid-Semihydrat (DE-PS 1 518 959 und 1 793 571) als brauchbar erwiesen hat. Es ist unter anderem in Form einer Lösung im Handel erhältlich. In dieser Schrift wird darauf hingewiesen, daß das Tilidinhydrochlorid-Semihydrat korrosiv an nicht besonders geschützten Werkzeugen wirkt.

Zur Tropfen-genauen Dosierung des hochwirksamen Analgetikums sind daher Pumpdosiereinrichtungen, wie eine Pumpe gemäß EP 0 014 754 A1 prinzipiell nicht einsetzbar, da diese metallische Oberflächen, beispielsweise die hier erwähnte metallische Feder oder Kugel, aufweisen. Aufgrund des korrosiven Verhaltens des Tilidins, insbesondere des Tilidinhydrochlorid in wässriger Lösung war ein an einen Einsatz derartiger Dosiervorrichtungen des Standes der Technik nicht zu denken.

Dementsprechend bestand die Aufgabe der vorliegenden Erfindung darin, an sich im-Stand der Technik bekannte wässrig/ethanolische Lösungen von Tilidin mit an sich im Stand der Technik bekannten Dosiervorrichtungen, die metallische Oberflächen aufweisende Pumpdosiereinrichtungen enthalten, miteinander kompatibel zu machen. Ein besonderes Problem stellen dabei die metallischen Oberflächen dar. Zwar sind im Stand der Technik eine Fülle von Pumpdosiereinrichtungen vorgeschlagen worden, die frei von metallischen Oberflächen sind und vollständig aus Kunststoffmaterialien bestehen, jedoch fehlt diesen Pumpdosiereinrichtungen, die Genauigkeit einer Tropfen-genauen Dosierung, die für ein hochwirksames Analgetikum wie Tilidin erforderlich ist.

Überraschenderweise wurde gefunden, daß bestimmte wässrig/ethanolische Lösungen von Tilidin kompatibel in Bezug auf die Korrosionseigenschaften mit Dosiervorrichtungen sind, obwohl diese metallische Oberflächen aufweisende Pumpdosiereinrichtungen enthalten.

Dementsprechend besteht der Kern der vorliegenden Erfindung in einer Dosiervorrichtung für wässrig/ethanolische Lösungen von Tilidin umfassend eine metallische Oberflächen aufweisende Pumpdosiereinrichtung 1 zur Tropfen-genauen Dosierung der Lösung aus einem Vorlagebehälter, wobei die Lösung Tilidin in Form eines Salzes in einer Menge von 30 bis 100 mg pro ml, Naloxon in Form eines Salzes in einer Menge von 2,5 bis 7 mg pro ml und Ethanol in einer Menge von wenigstens 5 Vol.-%, bezogen auf die Gesamtlösung bis zur Löslichkeitsgrenze des Tilidins bei Raumtemperatur, enthält.

Im Rahmen der Untersuchungen zu der vorliegenden Erfindung wurde gefunden, daß die obengenannte wässrig/ethanolische Lösung von Tilidin überraschenderweise nicht die im Stand der Technik beschriebenen korrosiven Eigenschaften gegenüber metallischen Oberflächen aufweist.

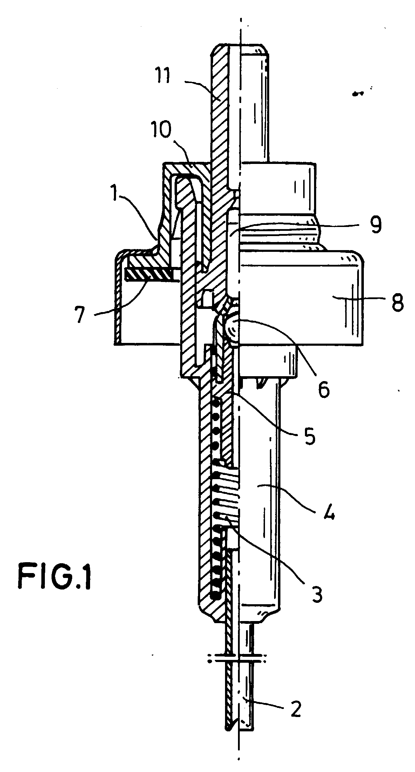

In der Fig. 1 wird eine geeignete Pumpdosiereinrichtung 1 beschrieben, die gemäß der vorliegenden Erfindung in vorteilhafterweise eingesetzt werden kann.

Die Fig. 1 zeigt eine Pumpdosiereinrichtung 1, bestehend aus einem Steigrohr 2, einer Feder 3, einem Kunststoffzylinder 4, einer kleinen Manschette 5, einer Kugel 6, einer Dichtung 7, einem Bördelring 8, einem Absperrstift 9, einem Schnappkörper 10 und großen Manschette 11.

Eine analoge, auch im Rahmen der vorliegenden Erfindung einsetzbare Pumpdosiervorrichtung ist der eingangs erwähnten EP 0 014 754 A1 zu entnehmen, auf die voll inhaltlich Bezug genommen wird.

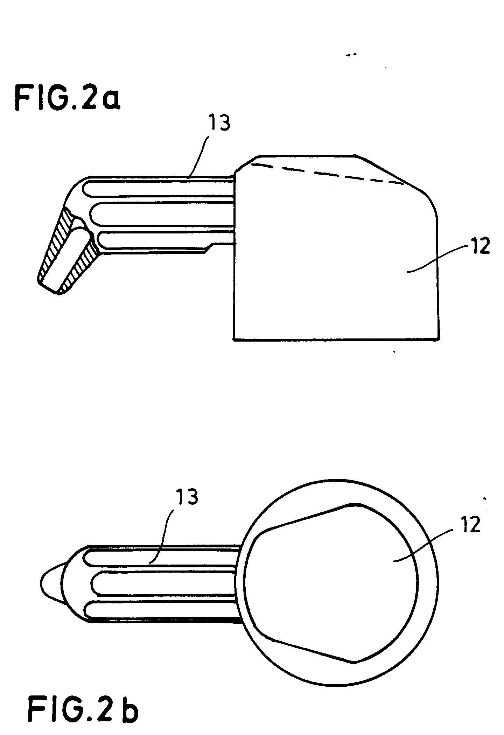

In der Fig. 2 wird ein Aufsatz für die Pumpdosiereinrichtung 1 beschrieben, der eine übliche Pumpdosiereinrichtung 1, die für Pumpen, insbesondere Zerstäuberpumpen geeignet ist, für die Tropfen-genaue Dosierung geeignet macht.

So ist es möglich, mittels einer durch den Beipackzettel des Medikaments vorgegebenen Zahl von Pumphüben eine Tropfen-genaue Dosierung vorzunehmen.

Die eingangs beschriebenen Dosiervorrichtungen sind auch dann zur Tropfen-genauen Dosierung von wässrig ethanolischen Lösungen von Tilidin geeignet, wenn die Feder 3 und/oder die Kugel 6 metallische Oberflächen, insbesondere aus Stahl aufweisen.

Erfindungsgemäß kann neben dem weit verbreitetem Hydrochlorid auch das Hydrogenfumarat, das Hydrogensulfat und/oder das Hydrogenphosphat eingesetzt werden. Es wurde gefunden, daß Korrosionsprobleme bei keinem der vorgenannten Salze in Lösung auftreten.

Naloxon, insbesondere Naloxon-Hydrochlorid wird üblicherweise den Tilidinzubereitungen als wirksamer Morphinantagonist zugesetzt. Erfindungsgemäß ergaben sich auch mit diesem Salz in Lösung keine Korrosionsprobleme.

Obwohl die Menge an Ethanol in der Lösung über einen sehr weiten Bereich variiert werden kann, ist es im Sinne der vorliegenden Erfindung besonders bevorzugt, den Alkoholgehalt möglichst niedrig einzustellen. Im Sinne der vorliegenden Erfindung wurde gefunden, daß Ethanolgehalte von 10 bis 15 Vol.-% ausreichen, um die Korrosions-fördernden Wirkungen des Tilidins zu beeinflussen.

Bei der an sich bekannten Korrosionswirkung des Tilidins kommt dem Schwermetallionengehalt der wässrigen ethanolischen Tilidinlösung eine gewisse Bedeutung zu. Erfindungsgemäß wurde gefunden, daß ein niedriger Schwermetallionengehalt eine verbesserte Korrosionswirkung ergibt. Dementsprechend besteht eine weitere bevorzugte Ausführungsform der vorliegenden Erfindung darin, daß die Lösung
Chrom in einer Menge von höchstens 0,1 mg/l,
Kupfer in einer Menge von höchstens 0,3 mg/l,
Nickel in einer Menge von höchstens 0,9 mg/l und
Eisen in einer Menge von höchstens 1,0 mg/l enthält.

Unter Einhaltung der o.g. Randbedingungen ist es möglich, eine wässrige ethanolische Lösung von Tilidin in einer entsprechenden Dosiervorrichtung anzubieten, die frei von Konservierungsmitteln ist.

## Patentansprüche

1. Dosiervorrichtung für wässrig/ethanolische Lösungen von Tilidin umfassend eine metallische Oberflächen aufweisende Pumpdosiereinrichtung (1) zur Tropfen-genauen Dosierung der Lösung aus einem Vorlagebehälter, wobei die Lösung Tilidin in Form eines Salzes in einer Menge von 30 bis 100 mg pro ml, Naloxon in Form eines Salzes in einer Menge von 2,5 bis 7 mg pro ml und Ethanol in einer Menge von wenigstens 5 Vol.-%, bezogen auf die Gesamtlösung bis zur Löslichkeitsgrenze des Tilidins bei Raumtemperatur, enthält.

2. Dosiervorrichtung für wässrig/ethanolische Lösungen von Tilidin nach Anspruch 1, wobei die Pumpdosiereinrichtung (1) ein Steigrohr (2), eine Feder (3), einen Kunststoffzylinder (4), eine kleine Manschette (5), eine Kugel (6), eine Dichtung (7), einen Bördelring (8), einen Absperrstift (9), einen Schnappkörper (10) und eine große Manschette (11) umfasst.

3. Dosiervorrichtung für wässrig/ethanolische Lösungen von Tilidin nach Anspruch 2, wobei die Feder (3) und/oder die Kugel (6) metallische Oberflächen insbesondere aus Edelstahl aufweisen,

4. Dosiervorrichtung für wässrig/ethanolische Lösungen von Tilidin nach Anspruch 2, wobei die Pumpdosiereinrichtung (1) weiterhin einen Sprühkopf (12) und eine Düse (13) aufweist.

5. Dosiervorrichtung für wässrig/ethanolische Lösungen von Tilidin nach Anspruch 1, wobei die Lösung Tilidin in Form des Hydrochlorids, des Hydrogenfumarats, des Hydrogensulfats und/oder des Hydrogenphosphats enthält.

6. Dosiervorrichtung für wässrig/ethanolische Lösungen von Tilidin nach Anspruch 1, wobei die Lösung Naloxon in Form des Hydrochlorids enthält.

7. Dosiervorrichtung für wässrig/ethanolische Lösungen von Tilidin nach Anspruch 1, wobei die Lösung 10 bis 15 Vol.-% Ethanol enthält.

8. Dosiervorrichtung für wässrig/ethanolische Lösungen von Tilidin nach Anspruch 1, wobei die Lösung
Chrom in einer Menge von höchstens 0,1 mg/l,
Kupfer in einer Menge von höchstens 0,3 mg/l,
Nickel in einer Menge von höchstens 0,9 mg/l und
Eisen in einer Menge von höchstens 1,0 mg/l enthält.

9. Dosiervorrichtung für wässrig/ethanolische Lösungen von Tilidin nach Anspruch 1, wobei die Lösung frei von Konservierungsmitteln ist.
